# EUROPÄISCHE PATENTSCHRIFT

(11) **EP 2 013 160 B1**
(45) Veröffentlichungstag und Bekanntmachung des Hinweises auf die Patenterteilung: **11.07.2012**
(21) Anmeldenummer: 07724141.2
(22) Anmeldetag: 11.04.2007
(51) Int. Cl.: C07C 68/08, A23L 2/44, C12H 1/14

(54) **STABILISIERUNG VON DIKOHLENSÄUREDIESTERN MIT PHOSPHOR-VERBINDUNGEN**
STABILIZATION OF DICARBONATE DIESTERS WITH PHOSPHORUS COMPOUNDS
STABILISATION DE DIESTERS D'ACIDE DICARBONIQUE COMPRENANT DES COMPOSÉS PHOSPHORÉS

(30) Priorität: 22.04.2006 DE 102006018843
(43) Veröffentlichungstag der Anmeldung: 14.01.2009
(73) Patentinhaber: LANXESS Deutschland GmbH, 51369 Leverkusen (DE)
(72) Erfinder: KAHLERT, Steffen, 42799 Leichlingen (DE); KAULEN, Johannes, 51519 Odenthal (DE); VOGL, Erasmus, 51373 Leverkusen (DE)
(86) Internationale Anmeldenummer: PCT/EP2007/003200
(87) Internationale Veröffentlichungsnummer: WO 2007/121857

(56) Entgegenhaltungen:
- WO-A-2005/123682
- US-A- 5 738 888
- DATABASE WPI Week 197144 Derwent Publications Ltd., London, GB; AN 1971-70699S XP002445031 & JP 46 037810 B (SUMITOMO CHEM CO LTD) 8. November 1971 (1971-11-08) in der Anmeldung erwähnt
- DATABASE WPI Week 197306 Derwent Publications Ltd., London, GB; AN 1973-07971U XP002445176 & JP 48 004016 B (HODOGAYA CHEM CO LTD) 5. Februar 1973 (1973-02-05) in der Anmeldung erwähnt

## Beschreibung

Die vorliegende Erfindung betrifft die Verwendung von Phosphor-Verbindungen als Stabilisatoren für Dikohlensäurediester, Mischungen enthaltend Dikohlensäurediester und Phosphor-Verbindungen sowie die Verwendung dieser Mischungen zur Konservierung von Lebensmitteln und Materialien.

Dikohlensäurediester werden unter anderem zur Konservierung von Lebensmitteln, insbesondere Getränken, als Bestandteile von antimikrobiellen Reagenzien, zur Deaktivierung von Enzymen in Fermentationsprozessen, oder zur Synthese von Feinchemikalien oder Polymeren verwendet. Dikohlensäurediester finden zudem z. B. als Katalysatoren zur Oxidation von Aminen oder zur Synthese, beispielsweise bei der Einführung von Schutzgruppen, Verwendung.

Es ist bekannt, dass die Stabilität von Dikohlensäurediestern bei Raumtemperatur und insbesondere bei erhöhter Temperatur relativ gering sein kann. Insbesondere während der Aufreinigung, z. B. bei einer destillativen Reinigung, oder während einer längeren Lagerung kann es daher zur Zersetzung von Dikohlensäurediestern kommen. Diese Zersetzung kann die Qualität und Reinheit der Dikohlensäurediester verschlechtern. Zudem schreitet die Zersetzung allgemein umso schneller voran, je mehr Verunreinigungen enthalten sind. Eine hohe Reinheit sowie eine Stabilisierung von Dikohlensäurediestern sind daher sehr erstrebenswert.

Aus dem Stand der Technik sind bereits Verfahren zur Verbesserung der thermischen Stabilität von Dikohlensäurediestern bekannt. So wird z. B. vorgeschlagen, Dialkylpyrocarbonate durch den Zusatz von Metallsulfaten zu stabilisieren (vgl. JP-A 48-4016). Nachteilig ist hierbei jedoch, dass diese Metallsulfate mit den Dikohlensäurediestern wenig bis schlecht mischbar sind.

Weiterhin ist bekannt, Dikohlensäurediester durch den Zusatz von Borverbindungen zu stabilisieren (vgl. JP-A 46-37810). Nachteilig ist hierbei jedoch besonders die Toxizität der entsprechenden Borverbindungen. Ein Einsatz in Lebensmitteln kommt für diese Zusätze nicht in Frage.

Weiterhin sind Carbonylverbindungen sowie heteroanaloge Carbonylverbindungen vorgeschlagen worden, als die Lagerstabilität erhöhende Zusatzmittel für Lösungen von Dikohlensäurediestern in gegenüber Dikohlensäurediestern inerten Lösungsmitteln (vgl. DE-A 3231397). Allerdings kommen Lösungen von Dikohlensäurediestern in üblichen aprotischen Lösungsmitteln kaum als Zusatz für Lebensmittel in Betracht. Zudem sind stabilisierende Effekte nur mit prozentual relativ großen Mengen an Zusätzen zu erreichen.

Es bestand daher Bedarf an Stabilisatoren, die geeignet sind Dikohlensäurediester wirkungsvoll gegen thermischen Zerfall zu schützen.

Uberraschenderweise wurde nun gefunden, dass Dikohlensäurediester durch Zusatz von Phosphor-Verbindungen gegen thermische und/oder chemische Abbaureaktionen, wie sie z.B. bei der Lagerung oder der Reinigung wie etwa der destillativen Reinigung auftreten können, stabilisiert werden können.

Gegenstand der vorliegenden Erfindung ist daher die Verwendung von mindestens einer PhosphorVerbindung zur Stabilisierung von Dikohlensäurediestern gegen chemische und/oder thermische Abbaureaktionen.

Bei den Dikohlensäurediestern handelt es sich bevorzugt um Verbindungen der allgemeinen Formel (I) 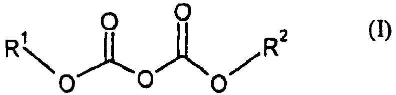 worin
- R¹ und R²: unabhängig voneinander für geradkettiges oder verzweigtes C₁-C₈-Alkyl, Cycloalkyl, C₂-C₈-Alkenyl, C₂-C₈-Alkinyl oder Benzyl stehen,
welches jeweils gegebenenfalls ein- bis mehrfach, gleich oder verschieden substituiert ist durch Halogen; Nitro; Cyano; C₁-C₆-Alkoxy; Dialkylamino; oder für Phenyl stehen, welches gegebenenfalls ein bis mehrfach, gleich oder verschieden substituiert ist durch Halogen; Nitro; Cyano; Alkyl; Halogenalkyl; Alkoxy; Halogenalkoxy; Acyl; Acyloxy; Alkoxycarbonyl; Carboxyl,
bevorzugt
- R¹ und R²: unabhängig voneinander für geradkettiges oder verzweigtes C₁-C₈-Alkyl, C₂-C₈-Alkenyl oder Benzyl stehen,
besonders bevorzugt
- R¹ und R²: unabhängig voneinander für geradkettiges oder verzweigtes C₁-C₅-Alkyl, C₃-Alkenyl oder Benzyl stehen,
und ganz besonders bevorzugt
- R¹ und R²: unabhängig voneinander für Methyl, Ethyl, Isopropyl, tert.-Butyl, tert.-Amyl, Allyl oder Benzyl stehen.

Bei den Stabilisatoren handelt es sich um Phosphor-Verbindungen aus der Reihe der Phosphoroxide und der Phosphor-Sauerstoff-Säuren und deren Derivate.

Beispielhaft seien als Phosphor-Sauerstoff-Säuren genannt: Ortho- und Metasäuren der allgemeinen Formeln H₃POₙ und HPOₙ₋₁ mit n = 2, 3, 4 und 5, Disäuren der allgemeinen Formel H₄P₂Oₙ mit n = 4, 5, 6, 7 und 8 sowie Pölyphosphorsäuren der allgemeinen Formel Hₙ₊₂PₙO₃ₙ₊₁ mit n = 3-15 000.

Als Derivate der Phosphor-Sauerstoff-Säuren sind insbesondere deren Salze und deren Ester zu nennen. Als Beispiele für Ester sind Mono-, Di- und Tri-Alkylester, Mono-, Di- und Tri-Alkenylester, Mono-, Di- und Tri-Arylester sowie Ester mit Zucker- oder Glycerinderivaten zu nennen. Bei den Alkylresten in den genannten Mono-, Di- und Trialkylestern handelt es sich beispielsweise um Methyl-, Ethyl-, n-Propyl-, iso-Propyl-, n-Butyl-, sec-Butyl-, tert-Butylreste. Die Ester können auch als Salze, z. B. Alkali- oder Erdalkalisalze, wie Natrium-, Kalium-, Magnesium-oder Calciumsalze vorliegen.

Als weitere Derivate der Phosphor-Sauerstoff-Säuren sind Verbindungen zu nennen, welche zudem mindestens eine Phosphor-Kohlenstoff Bindung enthalten. Beispiele für Phosphor-Kohlenstoff enthaltende Verbindungen dieser Art sind Derivate der Phosphonsäuren, Phosphonigen Säuren oder Phosphinsäuren und deren Ester. Als Beispiele für Ester sind Mono-, Di- und Tri-Alkylester, Mono-, Di- und Tri-Alkenylester, Mono-, Di- und Tri-Arylester sowie Ester mit Zucker- oder Glycerinderivaten zu nennen. Bei den Alkylresten in den genannten Mono-, Di- und Trialkylestern handelt es sich beispielsweise um Methyl-, Ethyl-, n-Propyl-, iso-Propyl-, n-Butyl-, sec-Butyl-, tert-Butylreste. Die Ester können ebenfalls als Salze , z. B. Alkali- oder Erdalkalisalze, wie Natrium-, Kalium-, Magnesium- oder Calciumsalze vorliegen.

Ganz besonders bevorzugt als Phosphor-Verbindungen sind Phosphorpentoxid (P₂O₅), Hypophosphorige Säure (H₃PO₂), Phosphorige Säure (H₃PO₃), wässrige oder kristalline Phosphorsäure (H₃PO₄), Pyrophosphorsäure, Metaphosphorsäure, Polyphosphorsäuren, Dimethylphosphat, Trimethylphosphat, Phosphate wie Natriumhydrogenphosphate oder Ammoniumhydrogenphosphate, Oleylphosphat, Phytinsäure, Phosphorylcholin, Adenosin-3'-monophosphorsäure, 2-Phosphonobutane-1,2,4-tricarbonsäure, Amino-trismethylen-phosphonsäure, Diethylenetriamin-pentamethylen-phosphonsäure oder Phosphonsäuregruppen-haltige Ionentauscher, wie sie z.B. bekannt sind aus WO 2000001458 oder aus EP-A 355007.

Die Phosphor-Verbindungen können dabei als Reinstoffe oder als wässrige oder alkoholische Lösungen eingesetzt werden. Ebenso können die Verbindungen in Pyrocarbonaten oder anderen geeigneten Lösungsmitteln vorgelöst werden. Die Phosphorverbindungen können auch auf Oberflächen, z. B. Glasoberflächen immobilisiert sein.

Zudem können selbstverständlich auch die unterschiedlichen reaktiven Phosphor-Halogenverbindungen, welche schon in Anwesenheit von nur geringen Mengen Wasser zu den oben genannten Phosphorverbindungen hydrolysieren, *in situ* eingesetzt werden. Beispiele hierfür sind Phosphortrichlorid oder Phosphorylchlorid..

Die genannten Stabilisatoren werden in einer Menge von 0.01 bis 3 000 ppm, bevorzugt in einer Menge von 0.1 bis 3000 ppm, besonders bevorzugt in einer Menge von 0.1 bis 2000 ppm bezogen auf den Dikohlensäurediester oder deren Gemisch eingesetzt.

Durch die erfindungsgemäße Verwendung gelingt es, Dikohlensäurediester generell gegen thermische und chemische Abbaureaktionen zu stabilisieren. Solche Abbaureaktionen treten z.B. bei der Lagerung auf.

Die erfindungsgemäß stabilisierten Dikohlensäurediester zeichnen sich durch eine verbesserte Lagerstabilität aus. So können die so stabilisierten Dikohlensäurediester über mehrere Monate bei Raumtemperatur gelagert werden, ohne dass eine Zersetzung der Dikohlensäurediester zu beobachten ist.

Weiterer Gegenstand der vorliegenden Erfindung sind Mischungen enthaltend einen oder mehrere Dikohlensäurediester der oben bezeichneten Formel (I) und eine oder mehrere der oben allgemein und bevorzugt beschriebenen Phosphor-Verbindungen in einer Menge von 0.01 bis 3 000 ppm, bevorzugt in einer Menge von 0.1 bis 3000 ppm, besonders bevorzugt in einer Menge von 0.1 bis 2000 ppm, bezogen auf den Dikohlensäurediester oder deren Gemisch. Ganz besonders bevorzugt sind Mischungen von mindestens einem Dikohlensäurediester der Formel (I), insbesondere von Dimethyldicarbonat und/oder Diethyldicarbonat mit einer oder mehreren Phosphor-Verbindungen aus der Reihe P₂O₅, H₃PO₂, H₃PO₃, wässrige oder kristalline H₃PO₄, Pyrophosphorsäure, Metaphosphorsäure, Polyphosphorsäuren, Dimethylphosphat, Trimethylphosphat, Phosphate wie Natriumhydrogenphosphate oder Ammoniumhydrogenphosphate, Oleylphosphat, Phytinsäure, Phosphorylcholin, Adenosin-3'-monophosphorsäure, 2-Phosphonobutane-1,2,4-tricarbonsäure, Amino-trismethylen-phosphonsäure, Diethylenetriamin-pentamethylen-phosphonsäure, Phosphortrichlorid, Phosphorylchlorid oder Phosphonsäuregruppen-haltige Ionentauscher.

Die erfindungsgemäßen Mischungen können über einen Zeitraum von mehreren Monaten gelagert werden, ohne dass es zu einer Zersetzung der darin enthaltenen Dikohlensäurediester kommt.

Die erfindungsgemäßen Mischungen eignen sich hervorragend zur Konservierung von Lebensmitteln und insbesondere Getränken gegen Befall und/oder Zersetzung durch Mikroorganismen, wie beispielsweise Bakterien, Pilze oder Hefen.

Ebenfalls Gegenstand der vorliegenden Erfindung ist die Verwendung der erfindungsgemäßen Mischungen zur Konservierung von Lebensmitteln und Getränken.

Die erfindungsgemäß stabilisierten Dikohlensäurediester eignen sich zum Beispiel in hervorragender Weise als Kaltentkeimungsmittel für stille oder karbonisierte Getränke wie Soft-Drinks, Vitamin-Getränke, Fruchtsaftgetränke, Teegetränke, alkoholische oder entalkoholisierte Wein-Getränke, Fruchtschorlen oder manche Biere. Üblicherweise werden dazu die Dikohlensäurediester in Mengen zwischen 10 und 250 ppm zeitnah zur Abfüllung den Getränken zugesetzt. Die Zumischung zu den Getränken erfolgt dabei mit speziellen Dosierpumpen. Die Dikohlensäurediester wirken dabei kontrollierend auf eine Reihe von Mikroorganismen wie fermentative Hefen, Schimmel oder fermentative Bakterien. Beispielhaft seien hier etwa genannt Saccharomyces cerevisiae, Mycoderma, Brettanomyces spp, Lactobacillus brevis, Lactobacillus buchneri und viele andere.

Thermische Abbaureaktionen von Dikohlensäurediestern treten des weiteren insbesondere auch bei der Aufarbeitung oder der Destillation von Dikohlensäurediestern auf, wie sie beispielsweise im Rahmen des Herstellungsverfahrens für Dikohlensäurediester durchgeführt wird. Durch die erfindungsgemäße Verwendung von Phosphor-Verbindungen gelingt es, Dikohlensäurediester mit geringeren Verlusten und in größerer Reinheit zu destillieren.

Weiterer Gegenstand der vorliegenden Erfindung ist daher ein Verfahren zur destillativen Reinigung von Dikohlensäurediestern, indem man einen oder mehrere Dikohlensäurediester der oben angegebenen Formel (I) mit einer oder mehreren der oben allgemein und bevorzugt beschriebenen Phosphor-Verbindungen, in einer Menge von 0.01 bis 3 000 ppm bezogen auf Dikohlensäurediester oder deren Gemisch, versetzt und anschließend die Mischung bei einem Druck von 5 bis 100 mbar, bevorzugt 10-70 mbar und einer Temperatur zwischen 30 und 120°C, bevorzugt zwischen 40 und 90°C, destilliert. Zur Destillation kommen die in der Technik üblichen Destillationskolonnen in Frage.

Die Ausbeuten an Dikohlensäurediester bei der Destillation liegen üblicherweise >99%.

Die folgenden Beispiele sollen den Gegenstand der Erfindung verdeutlichen, ohne diesen jedoch darauf zu beschränken.

### Beispiel 1

Entsprechend den Angaben in den Tabellen 1 - 6 wurden jeweils definierte Mengen eines bestimmten, hochreinen Dikohlensäurediesters und die jeweils angegebenen Zusätze in einem 10 ml Rundkolben mit Magnetrührer eingewogen. Die jeweils verwendeten, genauen Mengen der Zusätze sind ebenfalls den Tabellen zu entnehmen.

Der Rundkolben wurde mit einem Septum fest verschlossen. In diesem Septum befand sich eine Öffnung, worin ein Teflonschlauch befestigt war, der in eine senkrecht gestellte, mit Silikonöl gefüllte, auf 0.1 ml kalibrierte 50 ml Bürette geleitet wurde. An der Skalierung der Bürette konnte die Menge des sich durch die Zersetzung des Dikohlensäurediesters entwickelnden Kohlendioxids abgelesen werden. Der Kolben wurde zügig in ein, wie in den Tabellen 1 - 6 für den jeweiligen Versuch angegeben, konstant temperiertes Ölbad (gerührt mit 500 rpm) abgesenkt. Die Eintauchtiefe des Kolbens betrug 2,0 cm.

Nach der jeweils angegebenen Zeit, in der Regel nach 1, 2, 5, 10 und 15 Minuten, wurde das Gasvolumen abgelesen. Das Gasvolumen ist ein Maß für den Grad der Zersetzung der Dikohlensäurediester in CO₂. Es spiegelt damit umgekehrt den Grad der Stabilisierung durch die geprüften Zusätze wieder.

In den meisten Fällen wurden die Versuche wiederholt, um Reproduzierbarkeit sicherzustellen. Aussagekräftige Reproduzierbarkeit war jeweils gegeben.

Die Ergebnisse sind den Tabellen zu entnehmen. Hochreines Pyrocarbonat setzte in der beobachteten Zeit wenig Kohlendioxid frei, doch schon in Kontakt mit geringen Mengen von Silicagel, Braunstein oder auch nur rauhen Oberflächen, wie zerkratztem Glas, wurde die Zersetzung drastisch beschleunigt. Geringe Mengen der Stabilisatoren reichten aus um die Zersetzung effektiv zu verringern.

Je weniger gasförmige Zersetzungsprodukte Dikohlensäurediester unter Temperaturbelastung abgeben, desto günstiger wird eine Destillation unter Vakuum verlaufen.

**Diethyldicarbonat, 5000 ppm Zusatz Stabilisator (nicht erfindungsgemäß)**

| | | | | | | | |
|---|---|---|---|---|---|---|---|
| Temperatur [°C] Diethyldicarbonat | 130 | 130 | 130 | 130 | 130 | 130 | 130 |
| Menge [g] | 1 | 1 | 1 | 1 | 1 | 1 | 1 |
| Zusatz | ohne | Silikagel | Silikagel | Silikagel | Silikagel | Silikagel | Silikagel |
| Menge [mg] | | 10 | 10 | 10 | 10 | 10 | 10 |
| Zusatz Stabilisator | ohne | ohne | Al₂(SO₄)₃*18H₂O | krist. H₃PO₄ | 85%ige H₃PO₄ | Natriumsulfit | Na-4-Hydroxy-benzoat |
| Menge [mg] | | | 5 | 5 | 5 | 5 | 5 |

| **Gasentwicklung [ml]** | | | | | | | |
|---|---|---|---|---|---|---|---|
| Minuten 1 | 0,5 | 2,5 | 1,7 | 1,5 | 1,8 | 3,0 | 1,0 |
| Minuten 2 | 1,0 | 7,2 | 4,9 | 3,9 | 3,4 | 8,9 | 3,7 |
| Minuten 5 | 1,2 | 28,9 | 13,6 | 8,6 | 7,5 | 29,5 | 19,0 |
| Minuten 10 | 1,3 | 46,3 | 18,4 | 10,0 | 10,8 | 46,7 | 35,0 |
| Minuten 15 | 1,3 | 50,0 | 21,2 | 12,4 | 13,0 | 50,0 | 48,5 |

**Dimethyldicarbonat, 20 000 ppm Zusatz Stabilisator (nicht erfindungsgemäß)**

| | | | | |
|---|---|---|---|---|
| Temperatur [°C] | 100 | 100 | 100 | 100 |
| Dimethyldicarbonat Menge [g] | 1 | 1 | 1 | 1 |
| Zusatz | ohne | Silikagel | Silikagel | Silikagel |
| Menge [mg] | | 10 | 10 | 10 |
| Zusatz Stabilisator | ohne | ohne | krist. H₃PO₄ | 85%ige H₃PO₄ |
| Menge [mg] | | | 20 | 20 |

| **Gasentwicklung [ml]** | | | | |
|---|---|---|---|---|
| Minuten 1 | 0,5 | 1,3 | 0,7 | 0,1 |
| Minuten 2 | 1,4 | 3,9 | 1,7 | 0,3 |
| Minuten 5 | 3,3 | 20,1 | 4,5 | 2,3 |
| Minuten 10 | 5,7 | 49,0 | 10,8 | 9,6 |
| Minuten 15 | 8,8 | 50,0 | 16,7 | 14,4 |

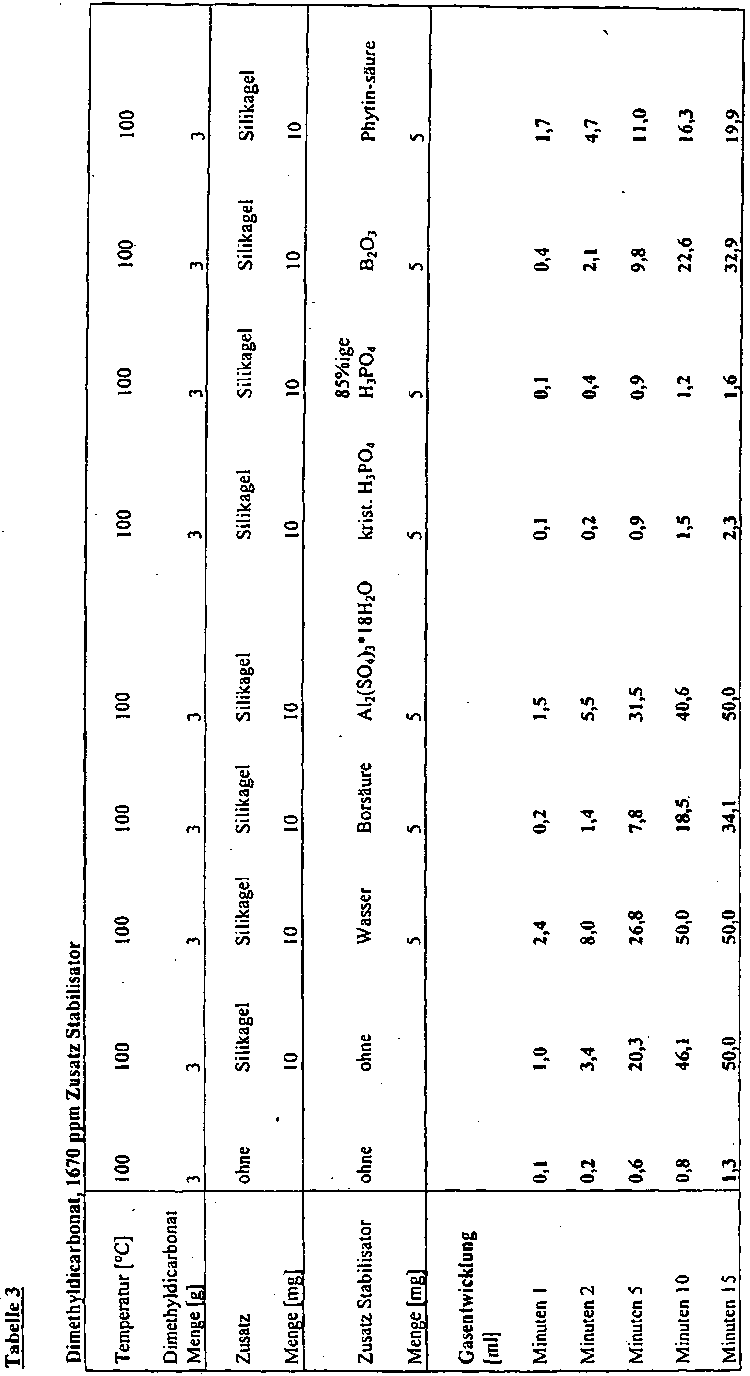

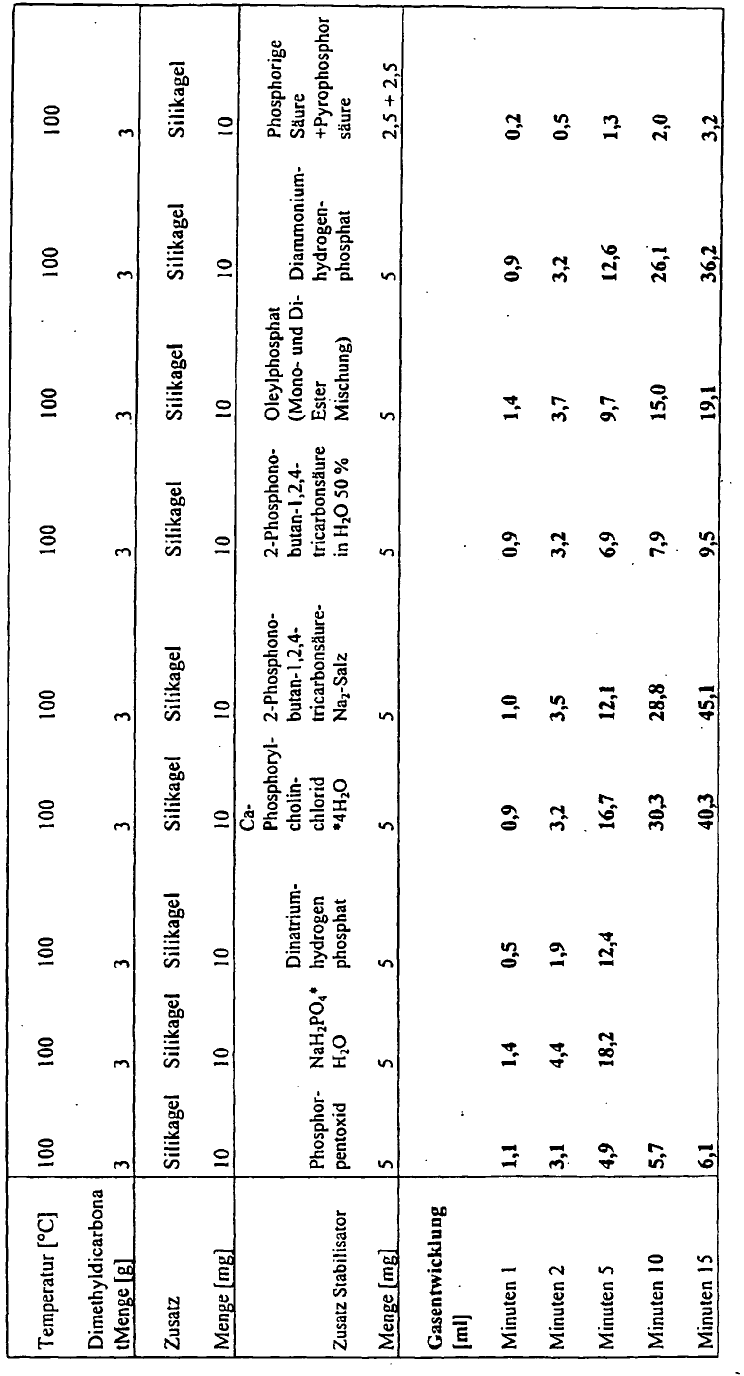

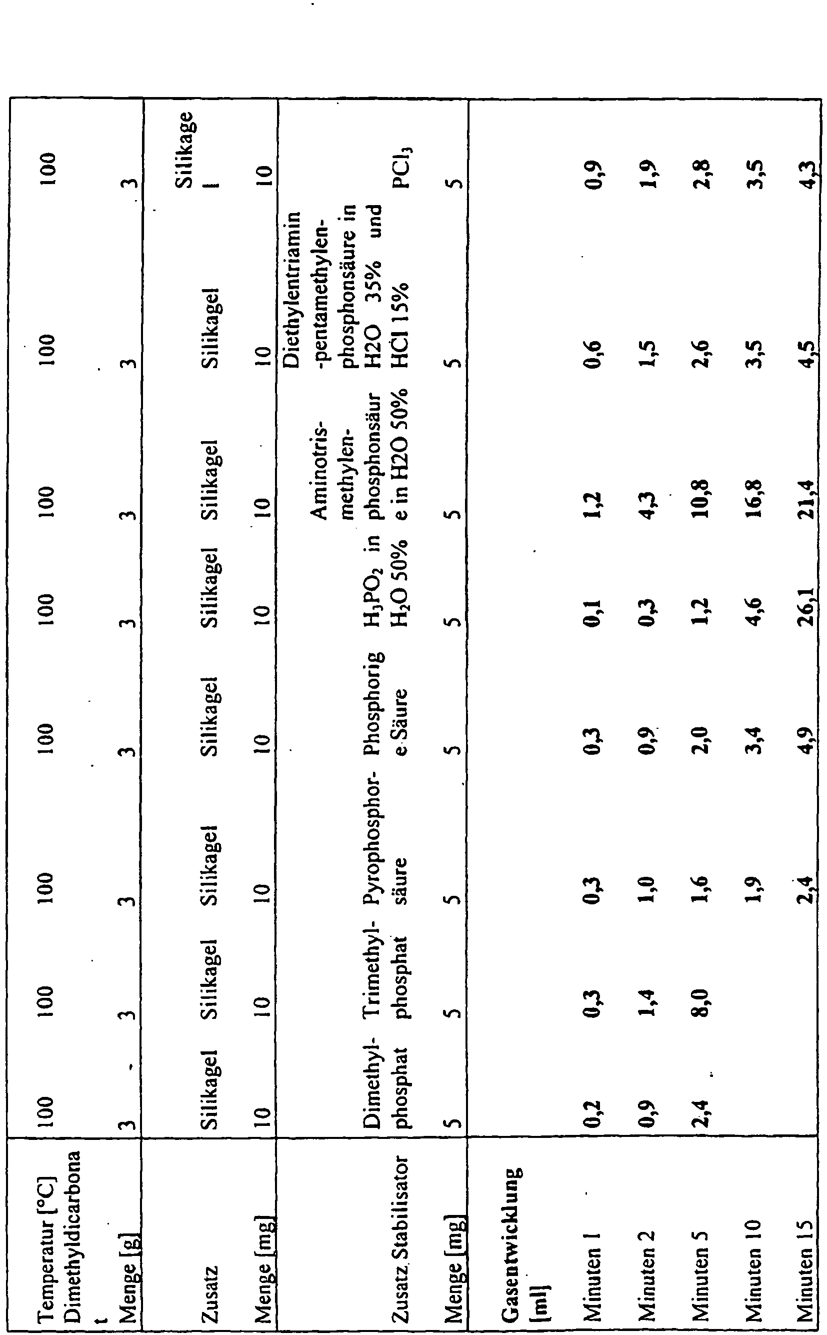

**Tabelle 4**

| **Dimethyldicarbonat, 1670 ppm Zusatz Stabilisator** | | | | | | | | |
|---|---|---|---|---|---|---|---|---|
| Temperatur [°C] | 100 | 100 | 100 | 100 | 100 | 100 | 100 | 100 |
| Dimethyldicarbonat Menge [g] | 3 | 3 | 3 | 3 | 3 | 3 | 3 | 3 |
| Zusatz | ohne | Braunstein | Braunstein | Braunstein | Braunstein | Braunstein | Braunstein | Braunstein |
| Menge [mg] | | 10 | 10 | 10 | 10 | 10 | 10 | 10 |
| Zusatz Stabilisator | ohne | ohne | krist. H₃PO₄ | 85%ige H₃PO₄ | Phosphor- pentoxid | 2-Phosphono-butan-1,2,4-tricarbonsäur e in H₂O 50 % | Diethylentriamin-pentamethylen-phosphonsäure in H2O 35% und HCl 15% | Wasser |
| Menge [mg] | | | 5 | 5 | 5 | 5 | 5 | 5 |

| **Gasentwicklung [ml]** | | | | | | | | |
|---|---|---|---|---|---|---|---|---|
| Minuten 1 | 0,1 | 3,8 | 0,2 | 1,0 | 0,6 | 1,4 | 1,5 | 7,1 |
| Minuten 2 | 0,2 | 9,3 | 0,8 | 2,1 | 1,5 | 3,9 | 3,1 | 26,4 |
| Minuten 5 | 0,6 | 21,7 | 1,4 | 3,9 | 2,5 | 8,2 | 6,8 | 35,4 |
| Minuten 10 | 0,8 | 31,1 | 1,8 | 5,4 | 2,9 | 11,4 | 9,7 | 46,1 |
| Minuten 15 | 1,3 | 41,5 | 2,3 | 6,4 | 3,4 | 12,9 | 12,1 | 50,0 |

**Tabelle 5**

| **Dimethyldicarbonat, 1670 ppm Zusatz Stabilisator** | | | |
|---|---|---|---|
| Temperatur [°C] | 100 | 100 | 100 |
| Dimethyldicarbona t Menge [g] | 3 | 3 | 3 |
| Zusatz | ohne | Oberfläche des Kolbens innen stark zerkratzt | Oberfläche des Kolbens innen stark zerkratzt |
| Zusatz Stabilisator | ohne | ohne | 85%ige H₃PO₄ |
| Menge [mg] | | | 5 |

| **Gasentwicklung [ml]** | | | |
|---|---|---|---|
| Minuten 1 | 0,1 | 4,0 | 1,7 |
| Minuten 2 | 0,2 | 6,0 | 2,6 |
| Minuten 5 | 0,6 | 7,6 | 3,4 |
| Minuten 10 | 0,8 | 10,0 | 4,8 |
| Minuten 15 | 1,3 | 11,0 | 5,5 |

**Tabelle 6**

| **Dimethyldicarbonat, <1000 ppm Zusatz Stabilisator** | | | | |
|---|---|---|---|---|
| Temperatur [°C] | 100 | 100 | 100 | 100 |
| Dimethyldicarbonat Menge [g] | 3 | 3 | 3 | 3 |
| Zusatz | Silikagel | Silikagel | Silikagel | Silikagel |
| Menge [mg] | 10 | 10 | 10 | 10 |
| Zusatz Stabilisator | 5 ppm 85%ige H₃PO₄ | 10 ppm 85%ige H₃PO₄ | 50 ppm 85%ige H₃PO₄ | 100 ppm 85%ige H₃PO₄ |

| **Gasentwicklung [ml]** | | | | |
|---|---|---|---|---|
| Minuten 1 | **1,5** | **0,7** | **1,1** | **1,3** |
| Minuten 2 | **4,0** | **1,9** | **2,2** | **2,6** |
| Minuten 5 | **14,3** | **6,8** | **5,3** | **4,9** |
| Minuten 10 | **32,2** | **25,0** | **8,2** | **6,5** |
| Minuten 15 | | **39,9** | **10,9** | **7,1** |

### Beispiel 2

Dimethyldicarbonat wurde bei Raumtemperatur gelagert. Als Maß für die Zersetzung wurde der Dimethylcarbonat-Gehalt per GC bestimmt. Ohne Zusatz eines Stabilisierungs-Mittels betrug der Dimethylcarbonat-Gehalt der Probe nach 3 Monaten 1090 ppm.

Der Versuch wurde wiederholt, jedoch wurden ca. 5 ppm Phosphorsäure zugesetzt. Die Versuchsreihe wurde mit 10 verschiedenen Proben durchgeführt. Der Dimethylcarbonat-Gehalt lag in diesem Fall nach 3 Monaten im Mittel bei lediglich 210 ppm.

## Patentansprüche

1. Verwendung von mindestens einer Phosphorverbindung aus der Reihe Phosphoroxide, Phosphor-Sauerstoff-Säuren und deren Derivate in einer Menge von 0.01 bis 3000 ppm bezogen auf Dikohlensäurediester oder deren Mischungen zur Stabilisierung von Dikohlensäurediestern gegen chemische und thermische Abbaureaktionen.

2. Verwendung gemäß Anspruch 1, **dadurch gekennzeichnet, dass** es sich bei den Phosphor-Sauerstoff-Säuren um Ortho- und Metasäuren der allgemeinen Formeln H₃POₙ und HPOₙ₋₁ mit n = 2, 3, 4 und 5, Disäuren der allgemeinen Formel H₄P₂Oₙ mit n = 4, 5, 6, 7 und 8 sowie Polyphosphorsäuren der allgemeinen Formel Hₙ₊₂PₙO₃ₙ₊₁ mit n = 3-15 000 handelt.

3. Verwendung gemäß wenigstens einem der Ansprüche 1 bis 2, **dadurch gekennzeichnet, dass** es sich bei den Dikohlensäurediestern um Verbindungen der allgemeinen Formel 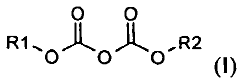 worin
R¹ und R² unabhängig voneinander für geradkettiges oder verzweigtes C₁-C₈-Alkyl, Cycloalkyl, C₂-C₈-Alkenyl, C₂-C₈-Alkinyl oder Benzyl stehen,
welches jeweils gegebenenfalls ein- bis mehrfach, gleich oder verschieden substituiert ist durch Halogen; Nitro; Cyano; C₁-C₆-Alkoxy; Dialkylamino; oder für Phenyl, welches gegebenenfalls ein bis mehrfach, gleich oder verschieden substituiert ist durch Halogen; Nitro; Cyano; Alkyl; Halogenalkyl; Alkoxy; Halogenalkoxy; Acyl; Acyloxy; Alkoxycarbonyl; Carboxyl; stehen,
handelt.

4. Verwendung gemäß wenigstens einem der Ansprüche 1 bis 3, **dadurch gekennzeichnet, dass** es sich bei den Dikohlensäurediestern um Dikohlensäuredimethylester oder Dikohlensäurediethylester handelt.

5. Verwendung gemäß wenigstens einem der Ansprüche 1 bis 4, **dadurch gekennzeichnet, dass** es sich um die Stabilisierung gegen Abbaureaktionen bei der Aufarbeitung, Extraktion, Destillation oder Lagerung handelt.

6. Mischungen enthaltend einen oder mehrere Dikohlensäurediester der allgemeinen Formel 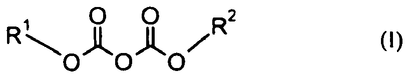 worin
R¹ und R² unabhängig voneinander für geradkettiges oder verzweigtes C₁-C₈-Alkyl, Cycloalkyl, C₂-C₈-Alkenyl, C₂-C₈-Alkinyl oder Benzyl stehen, welches jeweils gegebenenfalls ein- bis mehrfach, gleich oder verschieden substituiert ist durch Halogen; Nitro; Cyano; C₁-C₆-Alkoxy; Dialkylamino; oder für Phenyl, welches gegebenenfalls ein bis mehrfach, gleich oder verschieden substituiert ist durch Halogen; Nitro; Cyano; Alkyl; Halogenalkyl; Alkoxy; Halogenalkoxy; Acyl; Acyloxy; Alkoxycarbonyl; Carboxyl, stehen,
und eine oder mehrere Phosphor-Verbindungen aus der Reihe Phosphoroxide, Phosphor-Sauerstoff-Säuren und deren Derivate in einer Menge von 0.01 bis 3000 ppm bezogen auf Dikohlensäurediester oder deren Gemisch.

7. Mischung gemäß Anspruch 6, **dadurch gekennzeichnet, dass** sie mindestens eine Verbindung aus der Reihe Dimethyldicarbonat und Diethyldicarbonat und mindestens eine PhosphorVerbindung aus der Reihe P₂O₅, H₃PO₂, H₃PO₃, wässrige oder kristalline H₃PO₄, Pyrophosphorsäure, Metaphosphorsäure, Polyphosphorsäuren, Dimethylphosphat, Trimethylphosphat, Phosphate wie Natriumhydrogenphosphate oder Ammoniumhydrogenphosphate, Oleylphosphat, Phytinsäure, Phosphorylcholin, Adenosin-3'-monophosphorsäure, 2-Phosphonobutane-1,2,4-tricarbonsäure, Amino-trismethylen-phosphonsäure, Diethylenetriamin-pentamethylen-phosphonsäure, Phosphortrichlorid, Phosphorylchlorid oder Phosphonsäuregruppen-haltige Ionentauscher enthält.

8. Verwendung einer Mischung gemäß wenigstens einem der Ansprüche 6 bis 7 zur Konservierung von Lebensmitteln, Getränken und Materialien.

9. Verfahren zur destillativen Reinigung von Dikohlensäurediestern **dadurch gekennzeichnet, dass** man einen oder mehrere Dikohlensäurediester der allgemeinen Formel 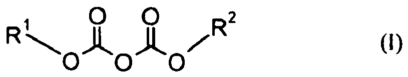 worin
R¹ und R² unabhängig voneinander für geradkettiges oder verzweigtes C₁-C₈-Alkyl, Cycloalkyl, C₂-C₈-Alkenyl, C₂-C₈-Alkinyl oder Benzyl stehen, welches jeweils gegebenenfalls ein- bis mehrfach, gleich oder verschieden substituiert ist durch Halogen; Nitro; Cyano; C₁-C₆-Alkoxy; Dialkylamino; oder für Phenyl, welches gegebenenfalls ein bis mehrfach, gleich oder verschieden substituiert ist durch Halogen; Nitro; Cyano; Alkyl; Halogenalkyl; Alkoxy; Halogenalkoxy; Acyl; Acyloxy; Alkoxycarbonyl; Carboxyl; stehen,
mit einer oder mehreren Phosphorverbindungen aus der Reihe Phosphoroxide, Phosphor-Sauerstoff-Säuren und deren Derivate in einer Menge von 0.01 bis 3000 ppm, bezogen auf Dikohlensäurediester oder deren Gemisch versetzt und anschließend die Mischung destilliert.

10. Verfahren gemäß Anspruch 9, **dadurch gekennzeichnet, dass** bei einem Druck von 5 bis 100 mbar und einer Temperatur zwischen 30 und 120°C destilliert wird.

## Claims

1. Use of at least one phosphorous compound from the series of phosphorus oxides, phosphorus-oxygen acids and derivatives thereof in an amount of 0.01 to 3 000 ppm, based on diesters of dicarbonic acid or mixture thereof for stabilization of diesters of dicarbonic acid against chemical and thermal breakdown reactions.

2. Use according to Claim 1, **characterized in that** the phosphorus-oxygen acids are ortho and meta acids of the general formulae H₃POₙ and HPOₙ₋₁ where n = 2, 3, 4 and 5, diacids of the general formula H₄P₂Oₙ where n = 4, 5, 6, 7 and 8, and also polyphosphoric acids of the general formula Hₙ₊₂PₙO₃ₙ₊₁ where n = 3-15 000.

3. Use according to at least one of Claims 1 to 2, **characterized in that** the diesters of dicarbonic acid are compounds of the general formula 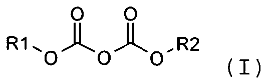 where
R¹ and R² independently of one another are straight-chain or branched C₁-C₈-alkyl, cycloalkyl, C₂-C₈-alkenyl, C₂-C₈-alkynyl or benzyl,
each of which is optionally monosubstituted to polysubstituted, identically or differently by halogen; nitro; cyano; C₁-C₆-alkoxy; dialkylamino; or are phenyl which is optionally monosubstituted to polysubstituted, identically or differently by halogen; nitro; cyano; alkyl; haloalkyl; alkoxy; haloalkoxy; acyl; acyloxy; alkoxycarbonyl; carboxyl.

4. Use according to at least one of Claims 1 to 3, **characterized in that** the diesters of dicarbonic acid are dimethyl dicarbonate or diethyl dicarbonate.

5. Use according to at least one of Claims 1 to 4, **characterized in that** the stabilization against breakdown reactions is during workup, extraction, distillation or storage.

6. Mixtures containing one or more diesters of dicarbonic acid of the general formula 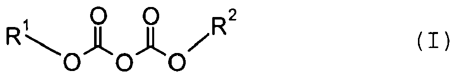 where
R¹ and R² independently of one another are straight-chain or branched C₁-C₈-alkyl, cycloalkyl, C₂-C₈-alkenyl, C₂-C₈-alkynyl or benzyl, each of which is optionally monosubstituted to polysubstituted, identically or differently by halogen; nitro; cyano; C₁-C₆-alkoxy; dialkylamino; or are phenyl which is optionally monosubstituted to polysubstituted, identically or differently by halogen; nitro; cyano; alkyl; haloalkyl; alkoxy; haloalkoxy; acyl; acyloxy; alkoxycarbonyl; carboxyl,
and one or more phosphorus compounds from the series of phosphorus oxides, phosphorus-oxygen acids and derivatives thereof in an amount of 0.01 to 3 000 ppm, based on diesters of dicarbonic acid or mixture thereof.

7. Mixture according to Claim 6, **characterized in that** it contains at least one compound from the series dimethyl dicarbonate and diethyl dicarbonate and at least one phosphorus compound from the series P₂O₅, H₃PO₂, H₃PO₃, aqueous or crystalline H₃PO₄, pyrophosphoric acid, metaphosphoric acid, polyphosphoric acids, dimethyl phosphate, trimethyl phosphate, phosphates such as sodium hydrogen phosphates or ammonium hydrogen phosphates, oleyl phosphate, phytic acid, phosphorylcholine, adenosine 3'-monophosphoric acid, 2-phosphonobutane-1,2,4-tricarboxylic acid, aminotrismethylenephosphonic acid, diethylenetriaminepentamethylenephosphonic acid, phosphorus trichloride, phosphoryl chloride or phosphonic-acid-group-containing ion exchangers.

8. Use of a mixture according to at least one of Claims 6 to 7 for preservation of foods, drinks and materials.

9. Method for the purification by distillation of diesters of dicarbonic acid, **characterized in that** one or more diesters of dicarbonic acid of the general formula 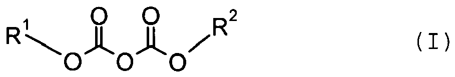 where
R¹ and R² independently of one another are straight-chain or branched C₁-C₈-alkyl, cycloalkyl, C₂-C₈-alkenyl, C₂-C₈-alkynyl or benzyl, each of which is optionally monosubstituted to polysubstituted, identically or differently by halogen; nitro; cyano; C₁-C₆-alkoxy; dialkylamino; or are phenyl which is optionally monosubstituted to polysubstituted, identically or differently by halogen; nitro; cyano; alkyl; haloalkyl; alkoxy; haloalkoxy; acyl; acyloxy; alkoxycarbonyl; carboxyl,
is admixed with one or more phosphorus compounds from the series of phosphorus oxides, phosphorus-oxygen acids and derivatives thereof in an amount of 0.01 to 3000 ppm, based on diesters of dicarbonic acid or mixture thereof and the mixture is subsequently distilled.

10. Method according to Claim 9, **characterized in that** distillation is performed at a pressure of 5 to 100 mbar and a temperature between 30 and 120°C.

## Revendications

1. Utilisation d'au moins un composé de phosphore de la série constituée par les oxydes de phosphore, les acides phosphore-oxygène et leurs dérivés en une quantité de 0,01 à 3 000 ppm par rapport à des diesters de l'acide dicarbonique ou leurs mélanges pour la stabilisation de diesters de l'acide dicarbonique contre des réactions de décomposition chimiques et thermiques.

2. Utilisation selon la revendication 1, **caractérisée en ce que** les acides phosphore-oxygène sont des ortho-et méta-acides de formules générales H₃POₙ et HPOₙ₊₁ avec n = 2, 3, 4 et 5, des diacides de formule générale H₄P₂Oₙ avec n = 4, 5, 6, 7 et 8, ainsi que des acides polyphosphoriques de formule générale Hₙ+₂PₙO3ₙ₊₁ avec n = 3 à 15 000.

3. Utilisation selon au moins l'une quelconque des revendications 1 à 2, **caractérisée en ce que** les diesters de l'acide dicarbonique sont des composés de formule générale 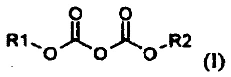 dans laquelle R¹ et R² représentent indépendamment l'un de l'autre alkyle en C₁-C₈ linéaire ou ramifié, cycloalkyle, alcényle en C₂-C₈, alcynyle en C₂-C₈ ou benzyle,
qui est à chaque fois éventuellement substitué une ou plusieurs fois, de manière identique ou différente, par halogène, nitro, cyano, alcoxy en C₁-C₆, dialkylamino ; ou phényle, qui est éventuellement substitué une ou plusieurs fois, de manière identique ou différente, par halogène, nitro, cyano, alkyle, halogénoalkyle, alcoxy, halogénoalcoxy, acyle, acyloxy, alcoxycarbonyle, carboxyle.

4. Utilisation selon au moins l'une quelconque des revendications 1 à 3, **caractérisée en ce que** les diesters de l'acide dicarbonique sont l'ester diméthylique de l'acide dicarbonique ou l'ester diéthylique de l'acide dicarbonique.

5. Utilisation selon au moins l'une quelconque des revendications 1 à 4, **caractérisée en ce qu'**il s'agit de la stabilisation contre des réactions de décomposition pendant le traitement, l'extraction, la distillation ou le stockage.

6. Mélanges contenant un ou plusieurs diesters de l'acide dicarbonique de formule générale 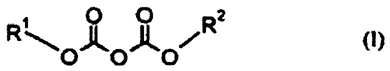 dans laquelle
R¹ et R² représentent indépendamment l'un de l'autre alkyle en C₁-C₈ linéaire ou ramifié, cycloalkyle, alcényle en C₂-C₈, alcynyle en C₂-C₈ ou benzyle, qui est à chaque fois éventuellement substitué une ou plusieurs fois, de manière identique ou différente, par halogène, nitro, cyano, alcoxy en C₁-C₆, dialkylamino ; ou phényle, qui est éventuellement substitué une ou plusieurs fois, de manière identique ou différente, par halogène, nitro, cyano, alkyle, halogénoalkyle, alcoxy, halogénoalcoxy, acyle, acyloxy, alcoxycarbonyle, carboxyle,
et un ou plusieurs composés de phosphore de la série constituée par les oxydes de phosphore, les acides phosphore-oxygène et leurs dérivés en une quantité de 0,01 à 3 000 ppm par rapport aux diesters de l'acide dicarbonique ou leur mélange.

7. Mélange selon la revendication 6, **caractérisé en ce qu'**il contient au moins un composé de la série constituée par le dicarbonate de diméthyle et le dicarbonate de diéthyle et au moins un composé de phosphore de la série constituée par P₂O₅, H₃PO₂, H₃PO₃, H₃PO₄ aqueux ou cristallin, l'acide pyrophosphorique, l'acide métaphosphorique, les acides polyphosphoriques, le phosphate de diméthyle, le phosphate de triméthyle, les phosphates tels que les hydrogénophosphates de sodium ou les hydrogénophosphates d'ammonium, le phosphate d'oléyle, l'acide phytique, la phosphorylcholine, l'acide adénosine-3'-monophosphorique, l'acide 2-phosphonobutane-1,2,4-tricarboxylique, l'acide amino-trisméthylène-phosphonique, l'acide diéthylènetriamine-pentaméthylène-phosphonique, le trichlorure de phosphore, le chlorure de phosphoryle ou les échangeurs d'ions contenant des groupes acide phosphonique.

8. Utilisation d'un mélange selon au moins l'une quelconque des revendications 6 à 7 pour la conservation de produits alimentaires, de boissons et de matériaux.

9. Procédé de purification distillative de diesters de l'acide dicarbonique, **caractérisé en ce qu'**un ou plusieurs diesters de l'acide dicarbonique de formule générale 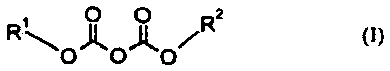 dans laquelle
R¹ et R² représentent indépendamment l'un de l'autre alkyle en C₁-C₈ linéaire ou ramifié, cycloalkyle, alcényle en C₂-C₈, alcynyle en C₂-C₈ ou benzyle, qui est à chaque fois éventuellement substitué une ou plusieurs fois, de manière identique ou différente, par halogène, nitro, cyano, alcoxy en C₁-C₆, dialkylamino ; ou phényle, qui est éventuellement substitué une ou plusieurs fois, de manière identique ou différente, par halogène, nitro, cyano, alkyle, halogénoalkyle, alcoxy, halogénoalcoxy, acyle, acyloxy, alcoxycarbonyle, carboxyle,
sont mélangés avec un ou plusieurs composés de phosphore de la série constituée par les oxydes de phosphore, les acides phosphore-oxygène et leurs dérivés en une quantité de 0,01 à 3 000 ppm par rapport aux diesters de l'acide dicarbonique ou leur mélange, puis le mélange est distillé.

10. Procédé selon la revendication 9, **caractérisé en ce que** la distillation est réalisée à une pression de 5 à 100 mbar et à une température comprise entre 30 et 120 °C.
